# DEMANDE DE BREVET EUROPEEN

(11) **EP 1 452 196 A1**
(43) Date de publication de la demande: **01.09.2004**
(21) Numéro de dépôt: 03405122.7
(22) Date de dépôt: 25.02.2003
(51) Int. Cl.: A61M 5/28

(54) **Dispositif et procédé de conditionnement d'un médicament**

(71) Demandeur: Rochat, Jean-Denis, 1272 Genolier (CH)
(72) Inventeur: Rochat, Jean-Denis, 1272 Genolier (CH)
(74) Mandataire: Savoye, Jean-Paul

(57) **Abrégé**

Ce dispositif comprend un corps creux en deux parties (1a, 1b) fixées hermétiquement l'une à l'autre, une première partie (1a) présentant successivement une cavité (3) pour un réservoir (3, 8), un siège de fixation étanche (5) pour une aiguille hypodermique (6) et un logement de protection (7) de la portion externe de cette aiguille (6), une membrane (13) pour former une barrière étanche entre ledit siège (5) et ledit réservoir (3, 8), ladite seconde partie (1b) présentant une première paroi (8) pour fermer ladite cavité (3), une seconde paroi déformable (9) solidaire de moyens (9a) pour perforer ladite membrane (13), une paroi intermédiaire pour former, entre ladite cavité (3) et ledit siège (5) un passage de liaison (14), un élément (10) pour fermer latéralement le siège de ladite aiguille hypodermique et une paroi de fermeture (11) dudit logement de protection (7).

## Description

La présente invention se rapporte à un dispositif jetable pour le conditionnement et l'injection d'une dose de médicament, comprenant un corps creux dans lequel sont ménagés un réservoir souple et étanche en plastique, chimiquement inerte vis-à-vis de ce médicament et un siège de fixation d'une aiguille hypodermique, ainsi qu'à un procédé pour le conditionnement d'un médicament liquide dans un dispositif d'injection jetable. Elle se rapporte également à un dispositif jetable pour la prise de sang.

La proportion de prises de sang et de médicaments injectés à très faibles volumes, typiquement entre 0,1 et 5 ml croît constamment, en raison de la sensibilité de plus en plus grande des dispositifs d'analyse ainsi que de la plus grande efficacité des médicaments injectés. Cette diminution de volume permet d'envisager de changer le mode opératoire que nécessite les seringues classiques et qui consiste à fixer une aiguille hypodermique stérile sur la seringue et à remplir la seringue de la quantité de médicament à injecter. Le temps de transfert du liquide à injecter, du flacon dans la seringue qui est d'environ 20 secondes, le risque de se piquer durant le remplissage de la seringue, le coût du flacon de médicament ainsi que celui des emballages de la seringue et de l'aiguille représentent une somme de frais et de dangers qui peuvent être évités.

On a déjà proposé depuis longtemps différentes solutions de dispositifs permettant le conditionnement d'un médicament liquide dans un réservoir souple en plastique chimiquement inerte auquel on fixe une aiguille hypodermique qui est séparée du liquide à injecter par un joint ou une fermeture susceptible de se rompre lors de l'application d'une pression suffisante sur le réservoir souple, permettant la distribution du médicament à travers l'aiguille. Un tel dispositif est décrit notamment dans le US 3'736'933.

Ce dispositif ne comporte cependant pas de protection de l'aiguille avant son utilisation, de sorte qu'un emballage de protection doit être prévu pour maintenir les aiguilles stériles jusqu'à leur utilisation.

On a déjà prévu dans le US 4'130'117 une ampoule d'injection munie d'une aiguille, une fermeture susceptible d'être rompue séparant le contenu de l'ampoule de l'aiguille et une coiffe soudée à l'ampoule protégeant l'aiguille jusqu'à son utilisation.

La production d'une telle ampoule doit être faite pièce par pièce. Aucune solution pour son remplissage n'est prévue, de sorte que la production d'un tel dispositif ne peut pas être obtenue sur des machines de conditionnement classiques, nécessitant le travail en continu à partir de matériau en bande, ou semi continu à partir de matériau en feuille destiné à la formation de plusieurs dispositifs d'injection disposés côte à côte.

La présente invention a pour but de remédier, au moins en partie, aux inconvénients des solutions existantes.

A cet effet, cette invention a pour objet un dispositif jetable pour le conditionnement et l'injection d'un médicament selon la revendication 1. Elle a également pour objet un procédé pour le conditionnement d'un médicament liquide dans un dispositif d'injection jetable selon la revendication 6. Elle a enfin pour objet un dispositif jetable pour prise de sang selon la revendication 7.

Par sa conception, ce dispositif comporte un logement de protection de l'aiguille hypodermique, permettant d'éviter tout autre emballage pour assurer la stérilité de l'aiguille, ce qui constitue un gain de coût non négligeable, ainsi qu'une garantie quant à la stérilité du dispositif.

De préférence, une amorce de rupture est formée entre le siège de l'aiguille et le logement de protection de cette aiguille, permettant une séparation aisée de la protection de l'aiguille du reste du dispositif lors de son utilisation.

Avantageusement, le volume ménagé entre la seconde paroi déformable et la membrane étanche séparant le réservoir de liquide de l'aiguille hypodermique est au moins égal au volume du conduit de cette aiguille, de sorte qu'en déformant la paroi déformable pour perforer la membrane, le liquide contenu dans le volume ménagé entre la seconde paroi déformable et la membrane étanche remplit le volume de l'aiguille hypodermique. Grâce à cette disposition, la rupture de la membrane met simultanément et automatiquement le dispositif d'injection en condition d'utilisation. Cela correspond à ce que fait le personnel médical avant toute injection lorsqu'il pousse le piston d'une seringue pour en chasser l'air jusqu'à ce qu'une goutte de liquide sorte à l'extrémité de l'aiguille, sans le risque de perdre une partie du médicament.

Outre les avantages susmentionnés du dispositif de conditionnement et d'injection selon la présente invention, la conception de ce dispositif permet une fabrication et un conditionnement des doses de médicaments à l'aide d'installations de conditionnement de types connus, à partir de matériaux en bandes, soit préalablement formés soit formés au cours du processus de conditionnement. Dans tous les cas les procédés de conditionnement utilisés sont habituels dans le domaine du conditionnement de produits à usage médical, permettant des cadences de production élevées dans les conditions d'asepsie requises.

D'autres particularités et avantages du dispositif selon la présente invention apparaîtront à la lumière de la description suivante qui sera faite à l'aide des dessins annexés qui illustrent, schématiquement et à titre d'exemple, une forme d'exécution du dispositif jetable pour le conditionnement et l'injection d'une dose de médicament, ainsi qu'un mode de mise en oeuvre du procédé de conditionnement, objets de la présente invention.
La figure 1 est une vue agrandie de dessus de cette forme d'exécution du dispositif;
la figure 2 est une vue en coupe selon la ligne II-II de la figure 1;
la figure 3 est une vue en plan montrant les différentes étapes de conditionnement d'une dose de médicament dans le dispositif selon la présente invention;
la figure 4 est une vue agrandie en coupe selon la ligne IV-IV de la figure 3 avant l'opération de soudage;
la figure 5 est une vue semblable à la figure 4 pendant l'opération de soudage.

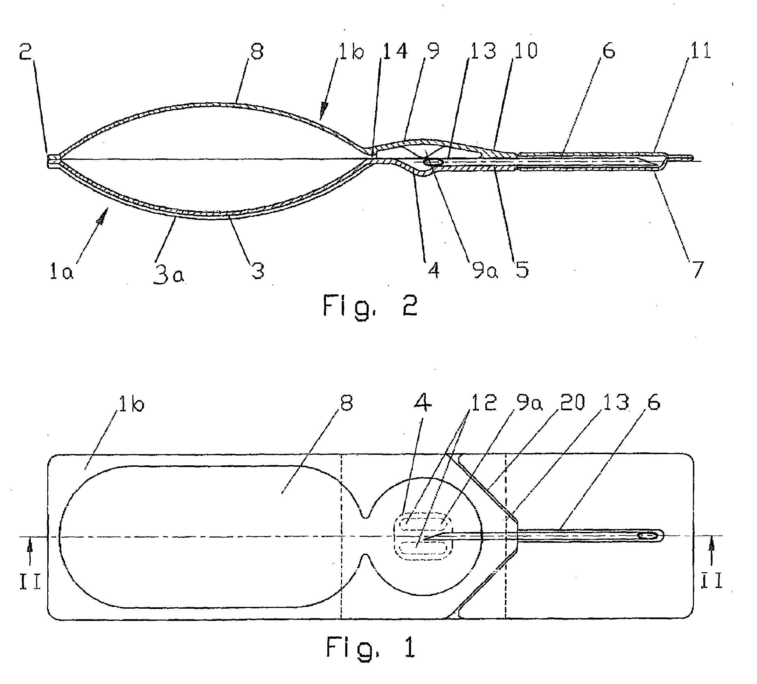

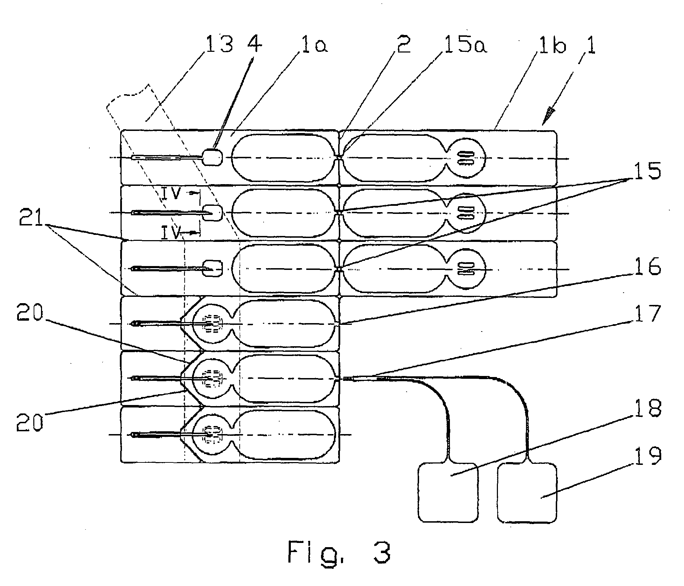

Le dispositif jetable pour le conditionnement et l'injection d'une dose de médicament illustré par les figures 1 et 2 résulte du pliage autour d'une ligne 2 (figure 3) et du soudage de deux parties, inférieure 1a, supérieure 1b d'une feuille 1, préalablement thermoformée, voire moulée par injection. En variante, les parties inférieure 1a et supérieure 1b peuvent être deux éléments séparés soudés l'un à l'autre plûtot qu'un seul élément plié autour de la ligne 2. La matière de la feuille 1 peut être en COC (copolymère cyclo-oléfine) ou un autre matériau thermoplastique inerte vis-à-vis du médicament à conditionner.

Le matériau en feuille 1 peut se présenter soit sous forme d'une bande continue, soit sous forme d'une feuille contenant un nombre déterminé de doses à injecter, par exemple 10 ou 12, séparées les unes des autres par des lignes de pré-découpage 21. Lorsque l'on part de feuilles séparées contenant un certain nombre de doses, on peut avantageusement conformer ces feuilles par moulage par injection plutôt que par thermoformage. Dans ce cas, il peut être avantageux de réaliser les parties inférieure 1a et supérieure 1b sous forme de feuilles séparées. Dans le cas d'une bande continue, celle-ci peut être séparée au fur et à mesure, entre des séries comportant un nombre désiré de doses restant attachées les unes aux autres par les lignes de pré-découpage 21.

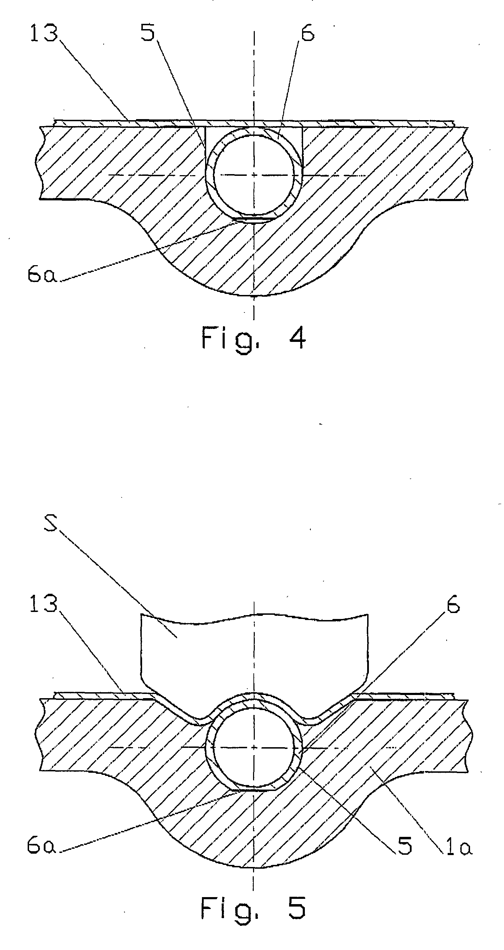

Lorsqu'on part d'un matériau en bande, avant d'être repliées l'une contre l'autre autour de la ligne de pliage 2, les parties inférieure 1a et supérieure 1b de la feuille 1 sont thermoformées pour ménager, dans la partie inférieure 1a une cavité 3 pour former au moins en partie un réservoir pour le médicament liquide à injecter. La paroi de cette cavité 3 présente, de préférence, des nervures 3a destinées à la rendre plus rigide. Cette cavité 3 est suivie par une petite empreinte 4 s'étendant de part et d'autre de l'axe longitudinal du dispositif et dont le rôle sera expliqué par la suite puis par un évidement 5 dont la section est en forme de U (voir figure 4), destiné à former un siège pour recevoir une aiguille hypodermique 6. Ensuite, la section de l'évidement 5 augmente pour ménager un logement allongé servant de protection 7 pour la partie de l'aiguille hypodermique 6 qui est située à l'extérieur du siège 5.

La partie supérieure 1b de la feuille 1 présente une cavité 8 symétrique à la cavité 3 et constituant l'autre paroi du réservoir pour la dose de médicament liquide. La paroi de cette cavité 8 ne présente pas de nervures et peut aussi être plus mince que celle de la cavité 3, notamment lorsque les deux cavités 3 et 8 sont réalisées à partir de deux feuilles distinctes, de manière à ce que, lorsqu'une pression est exercée sur les parois de ces cavités 3, 8, seule la paroi de la cavité 8 se déforme jusqu'à ce qu'elle épouse la forme de la cavité 3. La forme de cette cavité 3 et celle de la cavité 8 sont sensiblement symétriques et choisies pour qu'une fois que la paroi déformable de la cavité 8 est appliquée contre elle, cette paroi reste naturellement dans cette position déformée et est ainsi bi-stable, étant convexe dans une position et concave dans l'autre position, de sorte que la réutilisation de la seringue est rendue impossible, ce qui évite ainsi tout risque de contamination qui en résulterait.

Cette cavité 8 est suivie par une autre cavité 9 sensiblement plus petite, puis par une paroi 10 pour la fermeture du siège 5 de l'aiguille hypodermique 6 et enfin par une paroi 11 de fermeture du logement de protection 7 de l'aiguille 6. Deux saillies 9a sont formées à l'intérieur de la cavité 9. Elles ont un profil complémentaire à celui de l'évidement 4. Leur rôle sera expliqué par la suite. La portion de paroi séparant la cavité 8 de la partie supérieure 1b de la cavité 9 est conformée pour réserver un passage de communication 14 entre les deux cavités.

Comme on peut le voir sur la figure 2, une membrane étanche 13 est soudée, de manière à recouvrir toute la zone de la partie inférieure 1a située entre le passage de communication 14 et le siège 5 de l'aiguille 6, de manière à former une séparation étanche entre le réservoir formé entre les parois des cavités 3, 8 et le siège 5 de l'aiguille 6, isolant cette dernière du liquide contenu dans ce réservoir et dans la cavité 9 jusqu'à l'injection de ce liquide.

La figure 4 illustre le siège 5 et l'aiguille 6 recouverts par le film étanche 13, montrant que l'étanchéité n'est pas assurée entre le film 13 et l'aiguille 6. La figure 5 montre le soudage de la feuille étanche 13 par une sonotrode S qui provoque la déformation du film 13 et le fluage de la matière thermoplastique de la partie 1a de la feuille 1 adjacente au siège 5, de manière à entourer l'aiguille 6 de façon étanche. Simultanément, la matière thermoplastique de la feuille 1a soumise à l'échauffement provoqué par la sonotrode S remplit l'espace situé entre le siège 5 et un plat 6a formé sur la face externe de l'aiguille 6, de manière à assurer l'ancrage de l'aiguille au siège 5.

Lors du soudage des deux parties 1a, 1b l'une contre l'autre, une sonotrode S similaire à celle représentée par la figure 4 sera utilisée pour déformer la paroi de fermeture 10 du siège 5 de l'aiguille hypodermique 6, de manière à déformer cette paroi 10 pour lui faire épouser la forme de la membrane 13, la souder à cette membrane 13 et assurer ainsi l'étanchéité du siège 5 autour de l'aiguille 6.

Comme illustré par la figure 3, lors du thermoformage de la feuille 1, une rainure 15 est ménagée entre les cavités 3 et 8. Une découpe 15a est ménagée au centre de la rainure 15, sur la ligne de pliage 2, de sorte que lorsque la partie supérieure 1b est pliée sur la partie inférieure 1a autour de la ligne de pliage 2, une ouverture 16 apparaît permettant de faire communiquer le réservoir formé entre les cavités 3 et 8 avec l'extérieur. Cette ouverture 16 sert à permettre l'introduction d'un élément tubulaire à double conduit 17 dans le réservoir. L'un des conduits de cet élément tubulaire 17 est relié à une source d'aspiration 18 destinée à retirer l'air du réservoir 3, 8, tandis que l'autre conduit est relié à une source de médicament liquide 19 qui est aspiré dans le réservoir 3, 8 et dans le volume ménagé entre la membrane 13 et la seconde cavité 9, par la dépression créée par l'aspiration de la source 18. La dernière opération consiste à souder les parties 1a, 1b à l'endroit de l'ouverture 16 pour fermer hermétiquement le réservoir 3, 8.

Avant le remplissage de ce réservoir, simultanément au pliage des deux parties 1a, 1b l'une sur l'autre autour de la ligne de pliage 2, on découpe dans les zones planes des deux parties assemblées 1a, 1b de part et d'autre de l'extrémité externe du siège 5, à la jonction entre ce siège 5 et le logement de protection 7 de l'aiguille 6, deux lignes 20, formant un angle aigu en direction du réservoir 3, 8 pour dégager l'extrémité avant de la seringue, comme illustré en particulier sur la figure 1. Ces deux lignes découpées 20 forment une amorce de rupture entre la portion du dispositif contenant le logement de protection 7 et celle qui contient le réservoir (3, 8).

Lors de l'utilisation d'une dose de médicament, on sépare un dispositif contenant cette dose le long d'une ligne de pré-découpage 21. On imprime ensuite une torsion entre les deux portions du dispositif de conditionnement et d'injection situées de part et d'autre des deux lignes découpées 20, qui permettent de rompre facilement par cisaillement, la matière qui relie la portion de ce dispositif comportant le logement de protection 7 à celle qui comporte la dose de médicament, pour dégager l'aiguille hypodermique 6. Ensuite, une pression sur la cavité 9 permet de perforer la membrane 13 à l'aide des saillies 9a. L'évidement 4 reçoit les saillies 9a, de sorte que le liquide qui se trouvait dans la cavité 9 est chassé dans le canal de l'aiguille 6. Le volume de la cavité 9 est choisi pour être légèrement supérieur à celui du canal de l'aiguille 6, de sorte qu'une petite goutte de liquide perlera à l'extrémité de l'aiguille 6 une fois le liquide chassé hors de la cavité 9, montrant à l'utilisateur que le dispositif est prêt pour l'injection.

L'injection proprement dite s'effectue en pressant sur les parois 3, 8 du réservoir jusqu'à ce que le liquide soit complètement expulsé de ce réservoir, après avoir introduit l'aiguille à l'endroit désiré du corps d'un patient ou d'un animal. Après utilisation, le dispositif est destiné à être jeté.

Selon une variante, le dispositif décrit peut être légèrement modifié pour pouvoir l'utiliser pour prélever du sang au lieu d'injecter une dose de médicament. A cet effet, ce dispositif ne comporte plus ni membrane 13, ni cavité 9. D'autre part, la paroi 8 est conformée pour ne présenter qu'une position stable et non deux, de manière à revenir toujours dans la position illustrée par la figure 2. Le reste du dispositif est le même que celui décrit précédemment.

Pour faire une prise de sang, on rompt la liaison entre la protection 6, 7 et le siège 5, 10 de l'aiguille 6, on écrase la paroi 8 contre la paroi 3, on introduit l'aiguille dans la veine du patient, et on relâche la pression sur la paroi de la cavité 8 pour lui permettre de revenir dans la position illustrée par la figure 2 en aspirant du sang.

## Revendications

1. Dispositif jetable pour le conditionnement et l'injection d'une dose de médicament, comprenant un corps creux dans lequel sont ménagés un réservoir souple et étanche (3, 8) en plastique, chimiquement inerte vis-à-vis de ce médicament et un siège de fixation étanche (5) d'une aiguille hypodermique (6), **caractérisé en ce que** ledit corps creux comprend deux parties (1a, 1b) fixées hermétiquement l'une à l'autre, une première partie (1a) présentant successivement une cavité (3) pour ledit réservoir (3, 8), ledit siège de fixation étanche (5) et un logement de protection (7) de la portion externe de cette aiguille (6), une membrane (13) pour former une barrière étanche entre ledit siège (5) et ledit réservoir (3, 8), ladite seconde partie (1b) présentant une première paroi (8) pour fermer ladite cavité (3), une seconde paroi déformable (9) solidaire de moyens (9a) pour perforer ladite membrane (13), une paroi intermédiaire pour former, entre ladite cavité (3) et ledit siège (5) un passage de liaison (14), un élément (10) pour fermer latéralement le siège de ladite aiguille hypodermique et une paroi de fermeture (11) dudit logement de protection (7).

2. Dispositif selon la revendication 1, dans lequel une amorce de rupture (20) est formée entre ledit siège (5) et ledit logement de protection (7).

3. Dispositif selon l'une des revendications précédentes, dans lequel le volume ménagé entre ladite seconde paroi déformable (9) et ladite membrane étanche (13) est au moins égal au volume du conduit de ladite aiguille hypodermique (6), de sorte qu'en déformant ladite seconde paroi déformable (9) pour perforer ladite membrane (13), le liquide contenu dans ledit volume ménagé entre cette seconde paroi déformable (9) et ladite membrane étanche (13) permet de remplir le volume de ladite aiguille hypodermique (6).

4. Dispositif selon l'une des revendications précédentes, dans lequel ladite aiguille hypodermique 6 comporte des moyens d'ancrage (6a) entre cette aiguille et ledit siège (5).

5. Dispositif selon l'une des revendications précédentes, dans lequel la paroi de l'une (3) des cavités formant ledit réservoir (3, 8) comporte des moyens (3a) destinés à augmenter sa rigidité, tandis que la paroi de l'autre desdites cavités est conformée pour occuper deux positions bistables, l'une dans laquelle elle est convexe, l'autre dans laquelle elle est concave.

6. Procédé de conditionnement d'un médicament liquide dans un dispositif d'injection jetable, **caractérisé en ce qu'**on forme une première partie (1a) d'un corps creux dans laquelle on ménage une cavité (3) formant une portion de réservoir (3, 8) pour ledit liquide, un siège (5) pour une aiguille hypodermique (6), suivie d'un logement de protection (7) de la partie de cette aiguille hypodermique (6) s'étendant à l'extérieur dudit siège (5), on forme une seconde partie (1b) dudit corps creux dans laquelle on ménage une première cavité (8) pour fermer ladite cavité (3), une seconde cavité (9) solidaire de moyens de perforation d'une membrane (9a), une paroi intermédiaire pour former un passage de liaison entre lesdites première et seconde cavités (8, 9), une paroi (10) pour fermer ledit siège (5) et une paroi terminale pour fermer ledit logement de protection (7), on positionne ladite aiguille (6) sur ledit siège (5), on isole hermétiquement ledit siège (5) de ladite seconde paroi (9) à l'aide d'une membrane étanche (13) on positionne et on fixe hermétiquement l'une à l'autre lesdites première et seconde parties (1a, 1b) pour former ledit corps creux en laissant une ouverture de remplissage (16), on place dans cette ouverture un double conduit (17) dont l'un est relié à une source d'aspiration (18) et l'autre à une source dudit médicament (19) pour retirer l'air dudit réservoir (3, 8) et de ladite cavité seconde (9) et le remplacer par ledit médicament et on ferme hermétiquement ladite ouverture (16).

7. Dispositif jetable pour effectuer une prise de sang, comprenant un corps creux dans lequel sont ménagés un réservoir souple et étanche (3, 8) en plastique, chimiquement inerte vis-à-vis du sang et un siège de fixation étanche (5) d'une aiguille hypodermique (6), **caractérisé en ce que** ledit corps creux comprend deux parties (1a, 1b) fixées hermétiquement l'une à l'autre, une première partie (1a) présentant successivement une cavité (3) pour ledit réservoir (3, 8), ledit siège de fixation étanche (5) et un logement de protection (7) de la portion externe de cette aiguille (6), ladite seconde partie (1b) présentant une première paroi (8) pour fermer ladite cavité (3), une paroi intermédiaire pour former, entre ladite cavité (3) et ledit siège (5) un passage de liaison (14), un élément (10) pour fermer latéralement le siège de ladite aiguille hypodermique et une paroi de fermeture (11) dudit logement de protection (7).
